# EUROPEAN PATENT APPLICATION

(11) **EP 1 797 866 A2**
(43) Date of publication of application: **20.06.2007**
(21) Application number: 06025015.6
(22) Date of filing: 04.12.2006
(51) Int. Cl.: A61K 8/65, A61K 9/48, A61Q 11/00, A61P 31/02

(54) **Oral hygiene formulations in the form of capsules**

(30) Priority: 06.12.2005 IT MI20052342
(71) Applicant: Perfetti Van Melle S.p.A., 20020 Lainate (IT)
(72) Inventor: Colle, Roberto, 20020 LAINATE (MI) (IT); Sarrica, Andrea, 20020 LAINATE (M) (IT)
(74) Representative: Minoja, Fabrizio

(57) **Abstract**

Oral hygiene formulations in the form of capsules made of gelatin or similar gelling substances suitable for the alimentary use, filled with an oil containing an antiseptic agent for the oral cavity.

## Description

The present invention relates to capsules made of gelatin or of similar gelling substances, suitable for the alimentary use, filled with an oil containing an antiseptic agent for the oral cavity.

Collutories comprising antiseptic agents such as quaternary ammonium salts, chlorhexidine and the like, usually in the form of solutions, for use as mouth washes and washes of the oral cavity for combating halitosis, preventing formation of bacterial dental plaque, for the preparation of or subsequently to dental surgery and in any event when oral hygiene has to be maintained or improved through a decrease in the bacterial load usually present on oro-pharynx mucous membranes, are known.

The contact time of the solution and hence of the antiseptic agent usually ranges from a few seconds to some minutes, for the antibacterial agent to exert its action without irritating mucous membranes. After this time, the user usually expels the solution to prevent any systemic effects which could give rise to undesired effects.

As a consequence, a container (e.g. a glass and the like) for dosing the solution to use as well as a place in which expel the collutory, once the time necessary for the desired antiseptic action has lapsed, are usually necessary.

In some circumstances, for example in public places or means of transport, the use of the collutory is therefore substantially impossible and has to be delayed until the above cited conditions and means are available.

A portable collutory, easy-to-use in any circumstance and condition in the same way as a candy, a capsule and the like, would therefore be highly desirable.

The present invention aims at solving said problem by means of capsules made of gelatin or of similar gelling substances suitable for the alimentary use, filled with an oil containing an antiseptic agent for the oral cavity.

The capsules of the invention consist of an outer shell, usually of substantially spheroidal shape, preferably consisting of gelatine, having a diameter approximately ranging from 2 to 10 mm, preferably approx. from 3 to 6 mm. Alternatively to gelatine, natural polymers, such as optionally modified cellulose or amides, pectins, carrageenins, gums, alginates, optionally in a mixture thereof, can be used. Most preferred is gelatin. The weight percentage of the shell impermeable to the filling liquid usually approx. ranges from 5 to 10%.

The capsule outer shell can further contain flavours, dyes and sweetening agents in order to optimize the organoleptic properties. The shell can be transparent or translucid and of various colours.

The oil contained inside the capsule comprises at least one antiseptic agent such as cetylpyridinium chloride, benzalkonium chloride, decalinium chloride, ethyl resorcinol, chlorhexidine or salts thereof, Triclosan (2,4,4'-trichloro-2'-hydroxydiphenyl ether), polyphenol compounds, in particular polyphenol extracts from green tea.

Particularly preferred are cetylpyridinium chloride and Triclosan, optionally together with green tea extracts.

The following table reports the weight % ranges and preferred weight % ranges of the antiseptic agents according to the invention. Percentages are on the total weight of the capsule.

| Name | Percentages | | Preferred percentages | |
|---|---|---|---|---|
| | da % | a % | da % | a % |
| Cetylpyridinium chloride (CPC) | 5 | 0.01 | 0.2 | 0.05 |
| Triclosan (2,4,4'-trichloro-2'- hydroxyphenyl ether) | 5 | 0.01 | 0.2 | 0.05 |
| Benzalkonium Chloride | 3 | 0.001 | 0.1 | 0.01 |
| Decalinium Chloride | 5 | 0.01 | 0.2 | 0.05 |
| Hexyl Resorcinol | 5 | 0.01 | 0.2 | 0.05 |
| Chlorhexidine digluconate | 5 | 0.01 | 0.2 | 0.05 |
| Green tea polyphenols | 7 | 0.01 | 0.5 | 0.1 |

The oil contained in the shell may also be added with sweetening agents or flavours. Examples of suitable sweetening agents comprise Acesulfame K, aspartame, saccharin, sucralose, in appropriate amounts to give the desired taste, usually in amounts ranging from 0.1 to 1% on the total weight of the capsule.

The diluent oils contained inside the capsule, which act as carriers for the antiseptic agent and any other components, are edible oils such as coconut oil, olive oil, soybean oil, sunflower oil and the like, optionally fractionated or esterified.

The capsules of the invention can be prepared with known techniques and conventional devices, such as those used for the production of soft-gelatin capsules or "softgels". A more detailed description of the various conventional techniques for use in the preparation of the capsules of the invention is reported, for example, in US 2005/0100640 A1.

The capsules of the invention can be conveniently placed in the user's mouth, also thanks to their small size, in any circumstance thus providing a portable, easy-to-use collutory with no need for water, glasses, dosers and the like and can optionally also be ingested, as the topical antiseptic agent content is low.

The small size and nature of the carrier allow the substantially immediate consumption, as the capsules explode in the mouth immediately releasing the content. The capsules of the invention avoid the need for both containers for disposing non-edible parts and long times for sucking the product, as is the case with conventional candies or lozenges. Moreover, the very small amount of active agent allows for a repeated use during the day, minimizing any risks of undesired effects, thus keeping the continuous control of the proliferating bacterial flora.

The following examples illustrate the invention in greater detail.

| Example 1 - Macrocapsules | |
|---|---|
| Total Ingredients | % |
| Flavour | 19.000 |
| CPC | 0.100 |
| Green tea extract (highly purified) | 0.100 |
| Edible Oil | 74.298 |
| Aspartame | 0.500 |
| Sucralose | 0.250 |
| Gelatin | 5.500 |
| Acesulfame K | 0.250 |
| E133 Brilliant Blue | 0.001 |
| E129 Allura Red | 0.001 |
| Total | 100.000 |

| Example 2 - Filling oil of Example 1 | |
|---|---|
| Flavour | 20.160 |
| CPC | 0.106 |
| Green tea extract (highly purified) | 0.106 |
| Edible Oil | 78.832 |
| Aspartame | 0.531 |
| Sucralose | 0.265 |
| Total | 100.000 |

| Example 3 - Macrocapsules | |
|---|---|
| Total Ingredients | % |
| Flavour | 15.000 |
| Triclosan | 0.100 |
| Edible Oil | 79.000 |
| Aspartame | 0.400 |
| Sucralose | 0.250 |
| Gelatin | 4.999 |
| Acesulfame K | 0.250 |
| E 104 yellow quinoline | 0.001 |
| Total | 100.000 |

| Example 4 - Filling oil of Example oil of Example 3 | |
|---|---|
| Flavour | 15.831 |
| Triclosan | 0.106 |
| Edible Oil | 83.377 |
| Aspartame | 0.422 |
| Sucralose | 0.264 |
| Total | 100.000 |

## Claims

1. Capsules made of gelatin or similar gelling substances suitable for the alimentary use filled with an oil containing an antiseptic agent for the oral cavity.

2. The capsules as claimed in claim 1 wherein the capsule outer shell consists of gelatin, optionally modified cellulose or amides, pectins, carrageenins, gums, alginates, optionally also in a mixture thereof.

3. The capsules as claimed in claim 2 in which the shell consists of gelatin.

4. The capsules as claimed in any one of claims 1 to 3, in which the weight percentage of the impermeable shell approximately ranges from 3 to 10% on the total filling liquid.

5. The capsules as claimed in any one of claims 1 to 4, having substantially spheroidal shape and a diameter ranging from 2 to 10 mm.

6. The capsules as claimed in any one of claims 1 to 5, in which the antiseptic agent is selected from cetylpyridinium chloride, benzalkonium chloride, decalinium chloride, ethyl resorcinol, chlorhexidine or salts thereof, Triclosan (2,4,4'-trichloro-2'-hydroxyphenyl ether), green tea polyphenol extracts, alone or in a combination thereof.

7. The capsules as claimed in claim 6 containing cetylpyridinium chloride or Triclosan and optionally green tea extracts.

8. The capsules as claimed in claim 7 in which cetylpyridinium chloride or Triclosan are present in weight percentages ranging from 0.01 to 5% on the capsule total weight.

9. The capsules as claimed in any one of claims 1 to 7, further containing in the outer shell and/or in the oil therein flavours, sweetening agents and dyes.
